# EUROPEAN PATENT SPECIFICATION

(11) **EP 1 551 366 B1**
(45) Date of publication and mention of the grant of the patent: **12.11.2014**
(21) Application number: 03707568.6
(22) Date of filing: 29.01.2003
(51) Int. Cl.: A01N 43/80, A01N 59/20, A01N 59/16, A01N 47/44, A01N 43/653, A01N 43/40, A01N 41/10, A01N 37/22, A01N 31/16, A01N 31/04, A01N 25/34, A01N 25/10

(54) **ANTIMICROBIAL MELAMINE RESIN AND PRODUCTS MADE THEREFROM**
ANTIMIKROBIELLES MELAMINHARZ UND DARAUS HERGESTELLTE PRODUKTE
RESINE DE MELAMINE ANTIMICROBIENNE ET PRODUITS FABRIQUES A PARTIR DE CETTE DERNIERE

(30) Priority: 23.07.2002 WO PCT/US02/23184
(43) Date of publication of application: 13.07.2005
(73) Proprietor: MICROBAN PRODUCTS COMPANY, Huntersville, NC 28078 (US)
(72) Inventor: HANRAHAN, William, D., Charlotte, NC 28216 (US); ONG, Ivan W., Charlotte, NC 28210 (US); PARIANO, Laurie, J., Charlotte, NC 28269 (US)
(74) Representative: Boult Wade Tennant
(86) International application number: PCT/US2003/002545
(87) International publication number: WO 2004/009055

(56) References cited:
- EP-A- 0 505 064
- WO-A-01/10217
- WO-A-02/15693
- WO-A-02/17716
- WO-A-02/32989
- JP-A- 6 198 607
- US-A- 6 071 542
- US-B1- 6 248 342
- US-B1- 6 248 342
- DATABASE WPI Section Ch, Week 200005 Derwent Publications Ltd., London, GB; Class A82, AN 2000-058218 XP002341539 & JP 11 315227 A (TOA GOSEI CHEM IND LTD) 16 November 1999 (1999-11-16)
- "DERWENT CPI REGISTRY COMPOUNDS, INSTRUCTION MANAUL No. 2E" January 1987 (1987-01), DERWENT PUBLICATIONS LTD. , LONDON, UK , XP002341525 * entry 0859 = Melamine *
- DATABASE WPI Section Ch, Week 198024 Derwent Publications Ltd., London, GB; Class A21, AN 1980-42466C XP002341540 & JP 55 059907 A (YOSHITOMI PHARM IND KK) 6 May 1980 (1980-05-06)
- DATABASE WPI Section Ch, Week 199606 Derwent Publications Ltd., London, GB; Class A21, AN 1996-054250 XP002341541 & JP 07 313935 A (NISSHIN STEEL CO LTD) 5 December 1995 (1995-12-05)

## Description

The present invention concerns an antimicrobial melamine resin useful in making decorative laminates particularly for countertops, flooring, tabletops, desktops. More particularly, the present invention concerns the addition to melamine resin of an antimicrobial agent, which is capable of quickly and uniformly dispersing within the resin and exhibits antimicrobial properties for the life of the melamine layer.

Melamine resins are well known and were first synthesized in 1834 by Liebig. Prior to about 1940, these resins were more a laboratory curiosity than a commercial product. Today, melamine resin is widely used for surface coatings and decorative laminates. Paper based laminates are sold under the trade names of Formica® and Micarta®. These types of coatings or decorative laminates are formed from photographic prints or decorative sculptured paper impregnated with melamine resin and placed on a core material and cured in a large press. The kraft paper impregnated with the melamine resin is very compatible with the melamine resin and costs less than multiple layers of impregnated paper.

Melamine resins have the hardest surface of any commercial material. This hard surface along with excellent grease and water resistance, low flammability, and clarity of the plastic has led to the usefulness of melamine formaldehyde as countertop material. These countertop materials are thin sheets composed of heavy paper or thin cardboard backing that is bonded to the backside of a patterned paper. The bonding material is usually phenolic, which may be foamed, but is not thick. The top layer of paper is impregnated with melamine formaldehyde. The laminate is heated and subjected to pressure to attain a complete cure. The material generally is between two to four millimeters thick, up to about two meters in width, and sold as a roll in lengths. The roll sheet is then adhesively bonded to a wooden base to make countertop, flooring, furniture, etc.

The melamine resin is compatible with a wide variety of fillers and has been used for many molded parts because of the hard surface that results. Many products such as molded dinnerware, plastic dishes, and cups are made from the melamine resin which is hard, stain-resistant, and relatively low cost.

Pergo^{®} flooring is a ready to use (no sanding and no urethaning) laminated flooring system having a top layer of melamine formaldehyde that is heated under pressure to fuse to a wood layer. The top layer is a wear layer that is space age tough and yet transparent so that it allows the wood grain to show through.

Although melamine resin, and its uses such as with Formica^{®} or dinnerware, etc., are well known to those skilled in the art, making melamine resin having antimicrobial properties is quite difficult.

Despite the difficulty, there is a need in the industry to create tabletops, countertops, dinnerware, and flooring, all of which could be used either in a residential or commercial setting. More specifically, a restaurant having Formica® tabletops, countertops in the kitchen, and flooring throughout the restaurant, all of which exhibit antimicrobial properties, would be desirable and would reduce the chances of contamination from salmonella, E. coli, and other bacteria and fungi. Melamine resins are very hard, producing surfaces from melamine resins that have permanent antimicrobial characteristics, as opposed to a topical treatment that only has a temporary and very short-lived duration, has heretofore been unknown. Additionally, certain classes of antimicrobial agents are compatible or react with the melamine such that they are incorporated into the resin and no longer exhibit antimicrobial properties.

Peroxide has been identified as an antifungal and mildew proofing agent suitable for melamine resins in Japan publication JP 10-119221 filed Oct. 16, 1996 by Yozo Shioda. The peroxide is added at a concentration of 0.1 to 0.8 parts per 100 parts melamine. A problem associated with peroxide is that it rapidly degrades when heated, therein having shortened sustained antimicrobial properties. Additionally, at higher concentrations, the peroxide shortens the bath life of the melamine.

Antibacterial phosphates in combination with silver, benzalkonium, cety piridinium and isopropylmethylphenol has been described for use with melamine resins in Japan publication JP 07-329265 filed Jun. 3, 1994 by Kazuaki Tajima et al. for use in decorative laminates. The antibacterial phosphate along with silver, benzalkonium, cety piridinium and isopropylmethylphenol is added to the overlay sheet or the printed sheet, and to the underlying kraft paper which is impregnated with phenolic resins. The phosphates require a secondary antimicrobial, in part because phosphates are a food source for a number of elemental plants and animals (bacteria and fungi). An antimicrobial system that does not employ phosphates would be preferred, and especially an antimicrobial system that does not provide a source of nutrition for the bacteria or fungi. Furthermore, a preferred system would be one in which the antimicrobial is concentrated in the overlay sheet.

Disclosed in the abstract of JP11315227 (Ono et al.) is an antimicrobial lamellar silicate coating material, wherein a non-surface silicate layer therein contains a mildewcide-essentially, the lamellar silicate acts as a carrier for a mildewcide. The mildew-resistant coating material is taught to be applicable to metal, plastic, or ceramic substrates.

U.S. Patent No. 6,248,342 discusses a high-pressure laminate containing an inorganic metat-containing composition.

The use of antimicrobial compounds in combination is disclosed in PCT Publication No. WO02/32989 (Ederle et al.).

Combinations of isothiazolinone-based antimicrobial agents with alternative biocides is discussed in the prior art: WO02/15693 and WO02/17716 (both to Antoni-Zimmermann et al.) (synergistic combinations); and EP Publication No. 0 505 064 A1 (to Sherba et al.) (synergistic combination). Combinations of propiconazole and N-alkyl heterocyclic compounds are discussed in WO01/10217 (to Oppong et al.).

The need for dinnerware, picnic plates, cups, etc. also having antimicrobial properties exists for basically the same reasons given above with respect to surface countertops, tabletops and flooring. To create a melamine based resin having permanent antimicrobial properties such that the antimicrobial agent is capable of migrating through the very hard tough melamine resin, or is incorporated into the resin in such a manner that as the hard surface is abraded through normal wear and tear, newly exposed antimicrobial agent continuously renews the antimicrobial properties.

The present invention provides a permanently antimicrobial decorative laminate, comprising:
an overlay sheet impregnated with a melamine resin containing an antimicrobial agent in a dispersion, wherein the antimicrobial agent is an isothiazolone based compound;
a printed paper sheet impregnated with a melamine formaldehyde; and
a high density core fibreboard impregnated with a phenolic or melamine resin.

There can thus be provided a combination of a permanent antimicrobial melamine resin comprising melamine and an antimicrobial agent that is substantially inert with respect to the melamine resin and is present within the melamine resin in an amount effective to provide antimicrobial properties.

Effective additional antimicrobial agents are preferably those that have a relatively low vapour pressure. Triclosan, which is a diphenyl ether (bis-phenyl) derivative, having the scientific name of 2,4,4'-trichloro-2'-hydroxydiphenyl ether, is a particularly efficacious agent. It has a vapour pressure of 4 x 10⁻⁴ mm at 20°C. On the other hand, ortho phenyl phenol, (a.k.a. OPP-CAS No. 90-43-7), which has a boiling point of ∼280°C, is generally considered too volatile in the instant invention, except in applications necessitating superior resistance to bio-fouling. The combination of OPP and triclosan have been found to exhibit synergistic effects, and in products having a melamine formaldehyde coating where anti-bio fouling properties is required, the use of OPP can be justified. Isothiazolone based compounds selected from 1,2-benzisothiazolin-3-one (CAS # 2634-33-5), N-butyl-1,2-benzisothiazolin-3-one,2octyl-is-othiazolone (CAS # 26530-20-1), 4,5-dichloro-2-N-octyl-3(2H)-isothiazolone (CAS # 64359-81-5), methyl-3(2H)-isothiazolone (CAS # 2682-20-4), and chloro-2-methyl-3 (2H)-isothiazolone (CAS # 26172-55-4) have been found to be efficacious antimicrobial agents. Additional suitable agents include diiodomethyl p-tolylsulfone, zinc and sodium pyrithiones, azoles such as propiconazoles, poly(hexamethylene biguanide) hydrochloride, 3,4,4'-trichlorocarbanilide, barium metaborate (H₂O)n and silver, copper and zinc in zeolite or amorphous glass powder. In the invention, the antimicrobial(s) are delivered as a fine divided powder diluted in a liquid forming a dispersion. The dispersion is admixed with the melamine formaldehyde resins prior to the application of the resins. The dispersion is 15% to 65% solids by weight, and commonly 50% by weight, of the active antimicrobial. Zinc oxide can be added to the dispersion to stabilize the antimicrobial, and in particular zinc pyrithiones. The weight percent addition of the antimicrobial is 0.1 to 5%, with a preferred range of 0.3 to 1.0% on the weight of the melamine resin.

In a typical application, such as forming a melamine formaldehyde based decorative laminate (i.e. flooring or countertop) the laminate is comprised of a high density core which is at least one layer of fiberboard, to which is adhered a melamine-formaldehyde impregnated printed paper sheet, and onto which is adhered a melamine-formaldehyde impregnated overlay sheet. The impregnated printed paper is patterned to have the desired design, which in the instant case, is for counters or simulated wood. Commonly, the printed paper sheet and the overlay sheet are pre-impregnated with the melamine formaldehyde coating at least several days prior to forming the laminate. The fiberboard is usually impregnated with a phenolic or melamine resin. The pre-impregnation of the printed paper and the overlay enables the melamine formaldehyde sufficient time to thoroughly wet-out the substrate, which in turn drives out any remaining entrapped air. The melamine-formaldehyde is usually applied as a compounded resin/water or resin/water/solvent(s) saturate. Solvent(s) that are commonly used in melamine formaldehyde compositions are usually rather complex mixtures or more than one solvent, and include aromatics such as toluene, xylene and Solvesso 150® an aromatic blend by Exxon; alcohols such as butanol, isobutanol, methanol, ethanol, isopropanol; esters such as cellosolve acetate, ethyl acetate isopropyl acetate; ketones such as isophorone, methyl ethyl ketone, acetone; alcohol amines such as dimethyl ethanol amine, dimethyl isopropanol amine; and ethers such as butyl cellosolve. The use of solvents generally is reserved for impregnating difficult to saturate paper. Certain grades of kraft paper are either too dense, or too hydrophobic or a combination of the two to be saturated to a sufficient level of pick up to achieve the desired properties with a pure water borne system. More open porous paper sheets can be saturated with water/ latex systems, which generally have the advantage of a lower raw material cost.

Compounding additives, in addition to the antimicrobial, include surface active agents such as wetting agents, surfactants, de-aeratants, and defoamers; anti-blocking agents, catalysts such as PTSA (para toluene sulfonic acid), MSA (methane sulfonic acid), oxalic acid, ammonium nitrate and ammonium chloride; fillers; pigments; dielectric modifiers; glossing agents; and dyes. Latent acid catalysts, such as those having a fugitive counter ion, like ammonium nitrate and ammonium chloride, are preferred where the storage time will be lengthy. Generally speaking the strong acid catalysts are only used in melamine formaldehyde systems where the melamine is highly methylated, such as hexamethoxymethylmelamine. The antimicrobial dispersion can be used with melamine, and alkylated melamines such as methylated melamines, butylated melamines, isobutylated melamines, and melamines containing imino resins such as methylated imino resins, butylated imino resins, isobutylated imino resins; and urea resins such as methylated urea resins, butylated urea resins, isobutylated urea resins formaldehyde resins; benzoguanamine resins and glycoluril resins.

Following saturation the carrier (e.g. water and / or solvent) is dried off leaving the sheet saturated with the compounded melamine formaldehyde amine. It has been found that the antimicrobial only need be added to the overlay to impart antimicrobial properties to the laminate. While it is anticipated that migration into the printed paper layer occurs, antimicrobial, and in particular bactericidal performance remains efficacious at the 0.1 wt. % on melamine level. The laminate is formed by combining the overlay, the printed paper and the fiberboard in a heated press. Typical cure times are 15 seconds to several minutes at 127 °C to 290 °C. Pressures are on the order of 6.895 to 10.342 MPa (1000 psi to 5000 psi).

Antimicrobial agents can also be added directly to melamine formaldehyde resins, the antimicrobial agent of the present invention is incorporated into the bath through which the kraft paper passes, for example.

Suitable additional antimicrobial agents for the present invention may be 2,4,4'trichloro-2'-hydroxydiphenyl ether (also known as triclosan); 2-phenylphenol; poly(hexamethylene biguanide) hydrochloride, 3,4,4'-trichlorocarbanilide, zinc omadine (derivatives of pyrithione), and zeolites containing copper, silver, and zinc. Isothiazolone based compounds are selected from the group consisting of 1,2-benzisothiazolin-3-one (CAS # 2634-33-5), N-butyl-1,2-benzisothiazolin-3-one,2-octyl-isothiazolone (CAS # 26530-20-1), 4,5-dichloro-2-N-octyl-3(2H)-isothiazolone (CAS # 64359-81-5), methyl-3(2H)-isothiazolone (CAS # 2682-20-4), and chloro-2-methyl-3(2H)-isothiazolone (CAS # 26172-55-4).. Because several of these antimicrobial agents are capable of reacting with the melamine formaldehyde resin, they must consequently first be blended such that they are virtually encapsulated in a carrier that is compatible with melamine formaldehyde. This carrier containing antimicrobial agent is added to the melamine formaldehyde either in the bath when making solid surface materials or within the resin itself when compressed.

The present invention pertains to permanently antimicrobial decorative laminate made from melamine resin having the antimicrobial agent incorporated therein. In the broadest sense, the present invention relates to an article, such as flooring, countertops, tabletops, and cutting boards, which exhibit permanent antimicrobial properties during the useful life of such articles.

Melamine (C₃H₆N₆) is a white crystalline solid. Combining it with formaldehyde results in the formation of a compound referred to as methylol derivative. With additional formaldehyde (CH₂O) the melamine reacts to form tri-, tetra-, penta-, and hexamethylol-melamine. While commercial melamine resins may be obtained without the use of catalyst, both heating and catalyst are used to speed polymerization and curing. The reaction of the melamine with the formaldehyde is a condensation reaction with water as a byproduct. The curing of melamine resins is quickened by the use of heat and acid catalyst, while the overall pH is still neutral or slightly alkaline. The addition of acid catalyst provides a source for protons, but the level of addition is generally kept lower than alkalinity produced by the amines. Additionally, the melamine formaldehyde resin may contain fillers, plasticizers, dielectric modifiers, pigments or dyes, and glossing agents. When the resin is molded, the temperature range is generally between 154-171°C and under pressure in a compression mold from 13.79 to 34.47 MPa (2000 to 5000 psi).

Decorative laminates are usually assembled with a core of several sheets of phenolic or melamine resin impregnated kraft paper. The core is surfaced with a melamine formaldehyde impregnated sheet, which is often printed with a decorative design. Finally, a thin melamine resin-impregnated overlay sheet is applied. The sheets are impregnated by being passed through a resin bath, and followed by controlled moisture drying.

The melamine formaldehyde containing the dispersion of antimicrobial agent is incorporated into the saturating bath through which the paper passes. The bath is recirculated to keep the dispersion equally distributed. A typical melamine formaldehyde saturant will have a viscosity of 300 to 600 centipoise. The percent solids is 55% to 58%. The pH is 9.3 to 9.8. The bath is long enough such that there is a dwell time of approximately 1 minute. The overlay sheet is a 43 lb (70 gsm) to 50 lb (80 gsm) sheet (279 m², 3000 sq ft ream), which is saturated with 64.5 lb (105 gsm) to 68 lb (110 gsm) of resin (dry). The weight percent addition of the antimicrobial is 0.1 to 5%, with a preferred range of 0.3 to 1.0 wt.% on the melamine resin. The target weight percent of dry triclosan on melamine is 0.75%. Following saturation, the sheet is partially dried removing most of the dilution water. A similar saturation process is used on the impregnated printed paper sheet. The overlay, the printed paper sheet and the high density core fiberboard are assembled and laminated in a press at pressures ranging from 6.895 to 10.342 MPa (1000 to 1500 psi) at temperatures ranging from 170 to 210°C with a preferred temperature of 190 °C. The resulting laminate is mar resistant to detergents, acids, alcohols, oils and greases, and has antimicrobial properties throughout its entire life.

In a variation on the above described first embodiment, a second high density core fiberboard is formed, which is impregnated with melamine resin, and assembled and laminated to the first embodiment.

In a variation on the above described second embodiment, a rubberized pad is assembled and laminated to the bottom of the second high density core fiberboard.

Generally during curing, formaldehyde is released from melamine formaldehyde resin. Formaldehyde itself is a good antibacterial agent. However, when the curing is completed, insufficient amounts of formaldehyde are released and with time the amount released is smaller and smaller such that it simply has no antimicrobial effect, particularly after about one year as a molded article or in a surface material. The present invention relates to the incorporation of an antimicrobial agent in the melamine formaldehyde resin such that it always has antimicrobial properties throughout its entire life. The incorporation is effected through the addition of a dispersion of the antimicrobial agent to the melamine formaldehyde resin. The melamine resin is coated / saturated onto an overlay sheet used in a laminate. Following coating, dilution water and / or solvent is dried off, leaving the overlay impregnated with uncured melamine resin containing at least one antimicrobial. The overlay is combined with other sheets under heat and pressure, to form the laminate. The antimicrobial agent, which is concentrated in the overlay of the laminate imparts protective antimicrobial properties to the laminate for the life of the melamine coating.

## Claims

1. A permanently antimicrobial decorative laminate, comprising:
an overlay sheet impregnated with a melamine resin containing an antimicrobial agent in a dispersion, wherein the antimicrobial agent is an isothiazolone based compound;
a printed paper sheet impregnated with a melamine formaldehyde; and
a high density core fibreboard impregnated with a phenolic or melamine resin.

2. A laminate according to claim 1 wherein the isothiazolone based compound is selected from 1,2-benzisothiazolin-3-one, N-butyl-1,2-benzisothiazolin-3-one,2octyl-is-othiazolone, 4,5-dichloro-2-N-octyl-3(2H)-isothiazolone, methyl-3(2H)-isothiazolone, and chloro-2-methyl-3 (2H)-isothiazolone.

3. A permanently antimicrobial decorative laminate according to claim 1 or claim 2, comprising:
at least one other antimicrobial agent that is a chemical selected from triclosan, ortho phenyl phenol, diiodomethyl p-tolylsulfone, zinc pyrithiones, sodium pyrithiones, azoles such as propiconazoles, poly(hexamethylene biguanide) hydrochloride, 3,4,4'-trichlorocarbanilide, and barium metaborate (H₂O)ₙ.

4. A laminate according to claim 3, wherein said other antimicrobial agent is triclosan.

5. A laminate according to any preceding claim, further comprising:
a second high density core fibreboard impregnated with a phenolic or melamine resin.

6. A laminate according to any preceding claim, wherein the weight percent addition of the first antimicrobial agent is 0.1 to 5% based on the weight of the melamine resin.

7. A laminate according to claim 6, wherein the weight percent addition of the antimicrobial agent, based on the weight of the melamine resin, is 0.3 to 1.0%.

8. A method of making a laminate according to any preceding claim wherein a high density fibreboard impregnated with a phenolic or melamine resin is laminated with a printed paper sheet impregnated with a melamine formaldehyde and an overlay sheet impregnated with a melamine resin containing an isothiazolone based in a dispersion as an antimicrobial agent, and wherein said laminate is cured in a press at pressures ranging from 6.895 to 10.342 Mpa (1000 to 1500 psi), at temperatures ranging from 170 to 210°C.

9. A method according to claim 8 wherein said cure temperature is 190°C.

## Patentansprüche

1. Dauerhaft antimikrobielles dekoratives Laminat, das aufweist:
einen Overlay-Bogen, der mit einem Melaminharz getränkt ist, das einen antimikrobiellen Wirkstoff in Dispersion enthält, wobei es sich bei dem antimikrobiellen Wirkstoff um eine Verbindung auf Basis von Isothiazolon handelt;
einen bedruckten Papierbogen, der mit einem MelaminFormaldehyd-Harz getränkt ist; und
einen hochdichten Faserplattenkern, der mit einem Phenol- oder Melamin-Harz getränkt ist.

2. Laminat nach Anspruch 1, wobei die Verbindung auf Isothiazolon-Basis unter 1,2-Benzisothiazolin-3-on, N-Butyl-1,2-benzisothiazolin-3-on, 2-Octyl-isothiazolon, 4,5-Dichlor-2-N-octyl-3(2H)-isothiazolon, Methyl-3(2H)-isothiazolon und Chlor-2-methyl-3(2H)-isothiazolon ausgewählt ist.

3. Dauerhaft antimikrobielles dekoratives Laminat nach Anspruch 1 oder Anspruch 2, das aufweist:
zumindest einen weiteren antimikrobiellen Wirkstoff, bei dem es sich um einen chemischen Stoff handelt, der unter Triclosan, ortho-Phenylphenol, Diiodmethyl-p-tolylsulfon, Zink-Pyrithione, Natrium-Pyrithione, Azole wie Propiconazole, Poly(hexamethylen-biguanid)-Hydrochlorid, 3,4,4'-Trichlorcarbanilid und Bariummetaborat (H₂O)ₙ ausgewählt ist.

4. Laminat nach Anspruch 3, wobei es sich bei dem weiteren antimikrobiellen Wirkstoff um Triclosan handelt.

5. Laminat nach einem der vorhergehenden Ansprüche, das ferner aufweist:
einen zweiten hochdichten Faserplattenkern, der mit einem Phenol- oder Melamin-Harz getränkt ist.

6. Laminat nach einem der vorhergehenden Ansprüche, wobei der erste antimikrobielle Wirkstoff in einer Menge von 0,1 bis 5 Gew.-%, bezogen auf das Gewicht des Melaminharzes, zugegeben wird.

7. Laminat nach Anspruch 6, wobei der erste antimikrobielle Wirkstoff in einer Menge von 0,3 bis 1,0 Gew.-%, bezogen auf das Gewicht des Melaminharzes, zugegeben wird.

8. Verfahren zur Herstellung eines Laminats nach einem der vorhergehenden Ansprüche, worin eine hochdichte Faserplatte, die mit einem Phenol- oder Melamin-Harz getränkt ist, mit einem bedruckten Papierbogen, der mit einem Melamin-FormaldehydHarz getränkt ist, und einem Overlay-Bogen laminiert ist, der mit einem Melaminharz getränkt ist, das als antimikrobiellen Wirkstoff eine Verbindung auf Isothiazolon-Basis in Dispersion enthält, wobei das Laminat in einer Presse bei Drücken im Bereich von 6,895 bis 10,342 MPa (1000 bis 1500 psi) und Temperaturen im Bereich von 170 bis 210 °C gehärtet wird.

9. Verfahren nach Anspruch 8, wobei die Aushärtungstemperatur 190 °C beträgt.

## Revendications

1. Stratifié décoratif ayant une activité antimicrobienne permanente, comprenant :
une feuille de mise en train imprégnée d'une résine de mélamine contenant un agent antimicrobien dans une dispersion, dans lequel l'agent antimicrobien est un composé à base d'isothiazolone ;
une feuille de papier imprimée imprégnée d'un formaldéhyde de mélamine ; et
un panneau de fibres à coeur haute densité imprégné d'une résine phénolique ou de mélamine.

2. Stratifié selon la revendication 1 dans lequel le composé à base d'isothiazolone est choisi parmi la 1,2-benzisothiazolin-3-one, la N-butyl-1,2-benzisothiazolin-3-one, la 2-octyl-isothiazolone, la 4,5-dichloro-2-N-octyl-3(2H)-isothiazolone, la méthyl-3(2H)-isothiazolone et la chloro-2-méthyl-3(2H)-isothiazolone.

3. Stratifié décoratif ayant une activité antimicrobienne permanente selon la revendication 1 ou la revendication 2, comprenant :
au moins un autre agent antimicrobien qui est une substance chimique choisie parmi le triclosan, l'orthophénylphénol, la diiodométhyl-p-tolylsulfone, les pyrithiones de zinc, les pyrithiones de sodium, les azoles tels que les propiconazoles, le chlorhydrate de poly(hexaméthylène biguanide), le 3,4,4'-trichlorocarbanilide et le métaborate de baryum (H₂O)ₙ.

4. Stratifié selon la revendication 3, dans lequel ledit autre agent antimicrobien est le triclosan.

5. Stratifié selon l'une quelconque des revendications précédentes, comprenant en outre :
un deuxième panneau de fibres à coeur haute densité imprégné d'une résine phénolique ou de mélamine.

6. Stratifié selon l'une quelconque des revendications précédentes, dans lequel l'addition en pour cent en poids du premier agent antimicrobien est de 0,1 à 5 % par rapport au poids de la résine de mélamine.

7. Stratifié selon la revendication 6, dans lequel l'addition en pour cent en poids de l'agent antimicrobien, par rapport au poids de la résine de mélamine, est de 0,3 à 1,0 %.

8. Procédé de fabrication d'un stratifié selon l'une quelconque des revendications précédentes dans lequel un panneau de fibres haute densité imprégné d'une résine phénolique ou de mélamine est stratifié avec une feuille de papier imprimée imprégnée d'un formaldéhyde de mélamine et une feuille de mise en train imprégnée d'une résine de mélamine contenant un composé à base d'isothiazolone dans une dispersion en tant qu'agent antimicrobien, et dans lequel ledit stratifié est durci dans une presse à des pressions situées dans la plage de 6,895 à 10,342 MPa (de 1 000 à 1 500 psi), à des températures situées dans la plage de 170 à 210 °C.

9. Procédé selon la revendication 8 dans lequel ladite température de durcissement est de 190 °C.
